# FASCICULE DE BREVET EUROPEEN

(11) **EP 4 410 356 B1**
(45) Date de publication et mention de la délivrance du brevet: **24.12.2025**
(21) Numéro de dépôt: 23305127.5
(22) Date de dépôt: 31.01.2023
(51) Int. Cl.: A61M 60/191, A61M 60/289, A61M 60/481, A61M 60/486, A61M 60/839

(54) **DISPOSITIF D'ASSISTANCE DU MUSCLE CARDIAQUE D'UN ETRE VIVANT**
VORRICHTUNG ZUR UNTERSTÜTZUNG DES HERZMUSKELS EINES LEBEWESENS
DEVICE FOR ASSISTING THE HEART MUSCLE OF A LIVING BEING

(43) Date de publication de la demande: 07.08.2024
(73) Titulaire: MDSP Tech-Lab, 31570 Sainte Foy d'Aigrefeuille (FR)
(72) Inventeur: PONCE, Daniel, 31570 Sainte Foy d'Aigrefeuille (FR); BARILLOT, François, 38240 Meylan (FR); REBUFA, Jocelyn, 38240 Meylan (FR); PAGES, Alexandre, 38240 Meylan (FR)
(74) Mandataire: Icosa

(56) Documents cités:
- WO-A1-98/55165
- FR-A1- 3 120 797
- US-A1- 2011 092 761

## Description

### DOMAINE DE LA DIVULGATION

La présente invention concerne un dispositif d'assistance du muscle cardiaque d'un être vivant.

### ÉTAT DE LA TECHNIQUE

L'invention s'applique tout particulièrement au traitement de l'insuffisance cardiaque, une maladie du muscle cardiaque ou myocarde.

L'insuffisance cardiaque est la première cause de mortalité dans les pays industrialisés. Un stade terminal de la maladie se traduit par une dilatation des cavités cardiaques ventriculaires, associée à une diminution des capacités de contraction des fibres musculaires qui deviennent de plus en plus faibles. Il en résulte une diminution du débit cardiaque et une altération des capacités du muscle cardiaque à assurer normalement son rôle de pompe, indispensable à la propulsion du sang dans l'organisme.

Pour traiter l'insuffisance cardiaque, la transplantation cardiaque est une solution à laquelle une infime proportion de patients en insuffisance cardiaque terminale a accès.

Une solution alternative consiste à recourir à un dispositif d'assistance circulatoire.

Des dispositifs d'assistance circulatoire connus reposent sur un remplacement complet du cœur par un cœur artificiel ou sur la mise en place d'une turbine aspirant le sang dans le ventricule gauche et l'éjectant dans l'aorte.

Toutefois, là encore, cette solution alternative n'est accessible qu'à une infime partie de patients en insuffisance cardiaque terminale. Elle n'offre, par ailleurs, pas entière satisfaction compte tenu des complications graves et de l'altération de la qualité de vie qu'elle peut engendrer. En particulier, lorsque le sang circule au contact d'un corps étranger, il coagule et forme des caillots pouvant migrer et provoquer des accidents emboliques (accidents vasculaires cérébraux, ischémie digestive ou de membre). Pour éviter la formation de caillot, il est alors indispensable de donner un traitement anticoagulant au long cours, exigeant une surveillance biologique rapprochée et lui-même pourvoyeur de complications hémorragiques. Les mécanismes d'autorégulation du muscle cardiaque ne sont plus possibles et les valves cardiaques sont remplacées par des prothèses nécessitant une anticoagulation en cas de prothèses mécaniques ou étant associée à des risques de dégénérescence pour les prothèses biologiques. La dimension diastolique de la fonction cardiaque (capacité d'adaptation aux variations de volémie et de remplissage des cavités cardiaques) n'est pas ou mal prise en compte. Par ailleurs, les dispositifs d'assistance circulatoire ne sont que partiellement implantables. Les contrôleurs électroniques et les sources d'énergie sont extérieurs au patient, impliquant une connexion de l'assistance à ces éléments par un câble traversant la peau du patient, ce qui est source d'infection pouvant conduire au décès.

D'autres dispositifs d'assistance circulatoire connus consistent en des systèmes de compression du muscle cardiaque agissant depuis sa surface extérieure, du type de ceux décrits dans les documents WO98/55165 A1 et US 6 464 655. De tels dispositifs d'assistance circulatoire permettent d'assister le muscle cardiaque tout en préservant les autres structures du cœur, et notamment le revêtement interne des cavités au contact du sang, les valves cardiaques ou encore les systèmes d'autorégulation nerveux (connexion du cœur au système nerveux autonome) et hormonaux.

Les systèmes de compression connus sont toutefois encombrants et donc difficilement implantables et présentent une efficacité limitée.

### RÉSUMÉ DE LA DIVULGATION

L'invention vise à résoudre les problèmes évoqués précédemment.

A cet effet, la divulgation concerne un dispositif d'assistance du muscle cardiaque d'un être vivant, le muscle cardiaque présentant un axe de muscle cardiaque entre un apex et une base, et une surface extérieure, le muscle cardiaque ayant un volume variable au cours d'un cycle cardiaque entre un volume diastolique et un volume systolique, le dispositif d'assistance comprenant :
- une enveloppe implantable présentant un axe d'enveloppe destiné à être placé selon l'axe de muscle cardiaque, l'enveloppe comportant une pluralité d'organes d'actionnement annulaires autour de l'axe d'enveloppe, chaque organe d'actionnement présentant une surface de contact configurée pour être placée en contact avec au moins une partie de la surface extérieure du muscle cardiaque, et
- un système de contrôle comprenant au moins un actionneur configuré pour déplacer les organes d'actionnement selon l'axe d'enveloppe de telle manière que l'enveloppe présente un état déployé dans lequel les organes d'actionnement sont éloignés les uns des autres et les surfaces de contact délimitent le volume diastolique du muscle cardiaque, et un état rétracté dans lequel les organes d'actionnement sont rapprochés les uns des autres et les surfaces de contact délimitent le volume systolique du muscle cardiaque.

Le dispositif d'assistance permet ainsi de réaliser une contention active pour assister, éventuellement amplifier et renforcer, des mouvements systoliques et diastoliques du cycle cardiaque du muscle cardiaque. Le dispositif d'assistance se présente sous la forme d'un exo-muscle implantable enveloppant le muscle cardiaque permettant ainsi d'en réduire l'encombrement. Les surfaces de contact peuvent en outre être réparties pour améliorer l'assistance des mouvements systoliques et diastoliques et mieux les adapter aux mouvements naturels du muscle cardiaque.

Les volumes diastolique et systolique peuvent être déterminés de toute manière appropriée. Il peut notamment s'agir des volumes diastolique et systolique naturels du muscle cardiaque du patient à un stade préalable à la mise en place du dispositif d'assistance et, le cas échéant, avant l'atteinte du muscle cardiaque par la pathologie. Il peut également s'agir de volumes diastolique et systolique moyens définis à partir d'une population appartenant à la même catégorie que le patient. Il peut bien entendu s'agir de tous autres volumes diastolique et systolique jugés appropriés par le personnel soignant.

Le muscle cardiaque peut présenter une pluralité de zones d'intérêt adjacentes selon l'axe de muscle cardiaque, chaque zone d'intérêt présentant une surface extérieure déplaçable selon un débattement entre une systole et une diastole du cycle cardiaque. La surface de contact de chacun des organes d'actionnement peut être configurée pour être placée en contact avec au moins une partie de la surface extérieure de l'une des zones d'intérêt, et ledit au moins un actionneur peut être configuré pour déplacer chacune des surfaces de contact selon une course correspondant à au moins une partie du débattement de la zone d'intérêt avec laquelle ladite surface de contact est en contact.

Tout comme les volumes diastolique et systolique, les débattements des différentes zones d'intérêt du muscle cardiaque peuvent être déterminés de toute manière appropriée. Il peut notamment s'agir de débattements naturels du muscle cardiaque du patient à un stade préalable à la mise en place du dispositif d'assistance et, le cas échéant, avant l'atteinte du muscle cardiaque par la pathologie. Il peut également s'agir de débattements moyens définis à partir d'une population appartenant à la même catégorie que le patient. Il peut bien entendu s'agir de tous autres débattements jugés appropriés par le personnel soignant.

Ces dispositions permettent de respecter la physionomie de la contraction du muscle cardiaque en appliquant des efforts répartis sur l'ensemble des zones d'intérêt selon les orientations spécifiques de chacune des zones d'intérêt du muscles cardiaques qui présentent une convergence permettant d'éjecter le volume de sang nécessaire.

La pluralité de zones d'intérêt du muscle cardiaque peut présenter au moins une zone d'intérêt apicale et une zone d'intérêt basale, le débattement de la zone d'intérêt apicale ayant une composante essentiellement axiale et le débattement de la zone d'intérêt basale ayant une composante essentiellement radiale. La pluralité d'organes d'actionnement peut alors comporter au moins un organe d'actionnement apical dont la surface de contact est configurée pour être placée en contact avec au moins une partie de la surface extérieure de la zone d'intérêt apicale, et un organe d'actionnement basal dont la surface de contact est configurée pour être placée en contact avec au moins une partie de la surface extérieure de la zone d'intérêt basale, la course de la surface de contact de l'organe d'actionnement apical étant essentiellement axiale selon au moins une partie de la composante essentiellement axiale du débattement de la zone d'intérêt apicale, la course de la surface de contact de l'organe d'actionnement basal étant essentiellement radiale selon au moins une partie de la composante essentiellement radiale du débattement de la zone d'intérêt basale.

Chaque organe d'actionnement peut comporter un corps annulaire autour de l'axe d'enveloppe et présentant une surface intérieure, et une pluralité de plaquettes réparties sur la surface intérieure du corps, les plaquettes présentant des surfaces élémentaires de contact opposées à la surface intérieure du corps, les surfaces élémentaires de contact des plaquettes formant la surface de contact de l'organe d'actionnement.

Ledit au moins un actionneur peut comprendre au moins un actionneur principal, notamment électromagnétique ou piézoélectrique, configuré pour déplacer les corps des organes d'actionnement les uns par rapport aux autres selon l'axe d'enveloppe.

La surface élémentaire de contact de chacune des plaquettes peut être déplaçable par rapport à la surface intérieure du corps de l'organe d'actionnement.

Chacune des plaquettes peut être montée sur la surface intérieure du corps de chacun des organes d'actionnement par l'intermédiaire d'un agencement de biellettes comprenant une première biellette présentant des extrémités opposées articulées respectivement sur ladite plaquette et sur ladite surface intérieure du corps de l'organe d'actionnement, et au moins une deuxième biellette présentant des extrémités opposées articulées respectivement sur ladite plaquette et sur la surface intérieure du corps de l'organe d'actionnement adjacent selon l'axe d'enveloppe, de sorte que le déplacement des organes d'actionnement selon l'axe d'enveloppe induise un déplacement de la plaquette par rapport à la surface intérieure du corps de l'organe d'actionnement.

Ledit au moins un actionneur peut comprendre au moins un actionneur local, notamment électromagnétique ou piézoélectrique, configuré pour déplacer la plaquette par rapport à la surface intérieure du corps de l'organe d'actionnement.

La pluralité d'organes d'actionnement peut comprendre au moins une paire d'organes d'actionnement comportant un organe d'actionnement externe et un organe d'actionnement interne, le corps de l'organe d'actionnement interne s'étendant en partie dans le corps de l'organe d'actionnement externe au moins dans l'état rétracté de l'enveloppe.

Selon des dispositions particulières, le dispositif d'assistance peut être synchronisé sur l'activité du cœur. L'activité mécanique peut ainsi s'associer à une synchronisation électrique pour obtenir une resynchronisation mécanique.

Le muscle cardiaque peut présenter une pluralité de paramètres d'activité. Le système de contrôle peut alors comprendre un dispositif de détection configuré pour détecter au moins l'un des paramètres d'activité et pour activer ledit au moins un actionneur à partir dudit au moins un paramètre d'activité détecté.

Ledit au moins un actionneur peut être activable électriquement et le système de contrôle peut comprendre une source d'alimentation en énergie électrique connectée audit au moins un actionneur.

La source d'alimentation en énergie électrique peut comprendre au moins une batterie implantable.

La divulgation propose également un procédé d'assistance du muscle cardiaque d'un être vivant, le muscle cardiaque présentant un axe de muscle cardiaque entre un apex et une base, et une surface extérieure, le muscle cardiaque ayant un volume variable au cours d'un cycle cardiaque entre un volume diastolique et un volume systolique, le procédé d'assistance mettant en œuvre le dispositif d'assistance tel que défini précédemment et prévoyant de :
- implanter l'enveloppe, la surface de contact de chacun des organes d' actionnement étant placée en contact avec au moins une partie d'une surface extérieure du muscle cardiaque, au moins une zone d'actionnement étant placée en regard d'au moins une zone d'intérêt du muscle cardiaque,
- déplacer les organes d'actionnement selon l'axe d'enveloppe entre l'état déployé de l'enveloppe dans lequel les organes d'actionnement sont éloignés les uns des autres et les surfaces de contact délimitent le volume diastolique du muscle cardiaque, et l'état rétracté de l'enveloppe dans lequel les organes d'actionnement sont rapprochés les uns des autres et les surfaces de contact délimitent le volume systolique du muscle cardiaque.

Le muscle cardiaque peut présenter une pluralité de zones d'intérêt adjacentes selon l'axe de muscle cardiaque, chaque zone d'intérêt présentant une surface extérieure déplaçable selon un débattement entre une systole et une diastole du cycle cardiaque. La surface de contact de chacun des organes d'actionnement peut être configurée pour être placée en contact avec au moins une partie de la surface extérieure de l'une des zones d'intérêt. Le procédé d'assistance peut alors prévoir de déplacer chacune des surfaces de contact selon une course correspondant à au moins une partie du débattement de la zone d'intérêt avec laquelle ladite surface de contact est en contact.

Le procédé d'assistance peut prévoir de détecter au moins un paramètre d'activité du muscle cardiaque et d'activer ledit au moins un actionneur à partir dudit au moins un paramètre d'activité détecté.

### DESCRIPTION DES FIGURES

D'autres objets et avantages de l'invention apparaîtront à la lecture de la description qui suit d'un mode de réalisation particulier de l'invention donné à titre purement illustratif et non-limitatif, la description étant faite en relation avec les dessins joints dans lesquels :

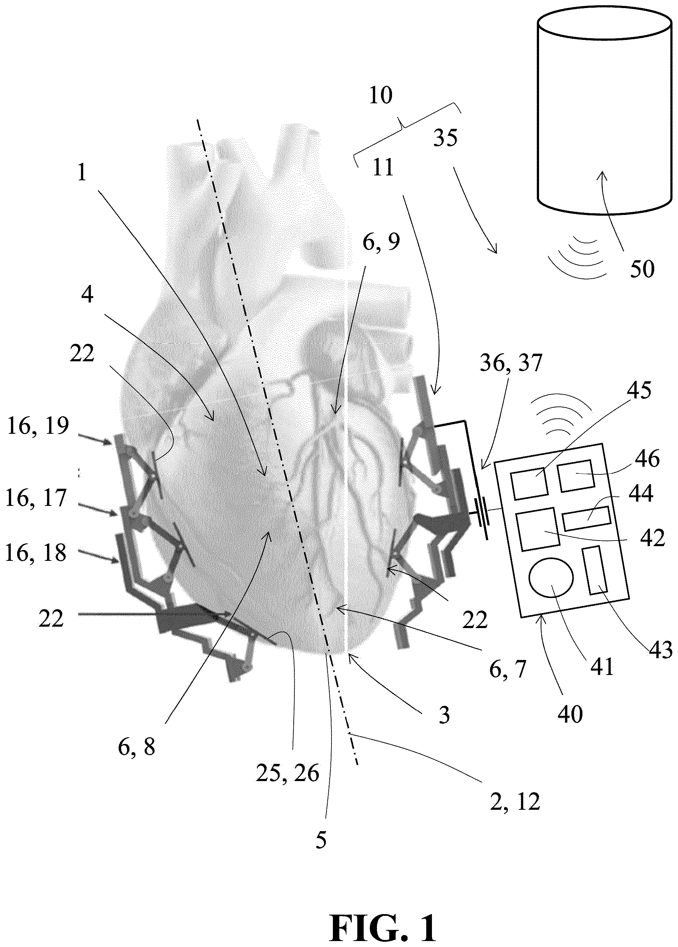

- la figure 1 représente un dispositif d'assistance configuré pour réaliser une contention active du muscle cardiaque, le dispositif d'assistance comprenant une enveloppe implantable configurée pour être placée en contact avec au moins une partie d'une surface extérieure du muscle cardiaque, et un système de contrôle,

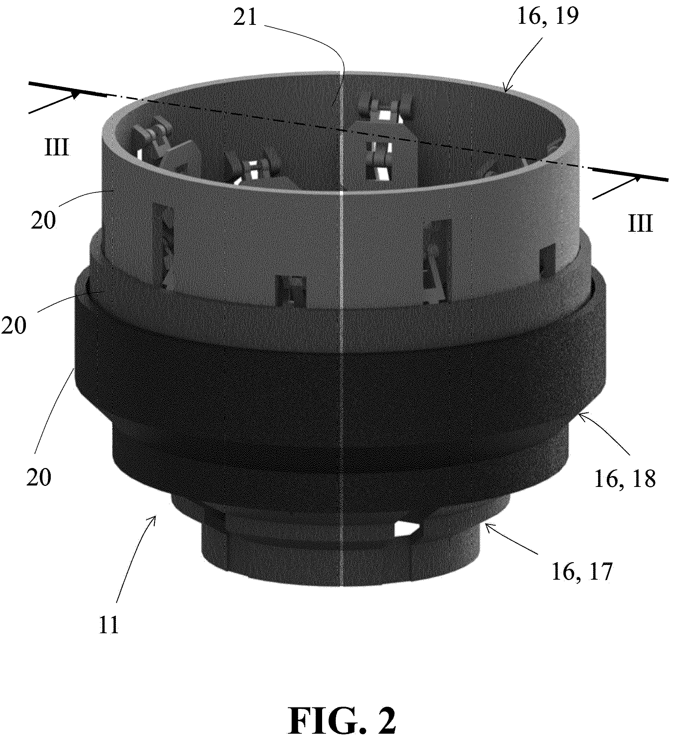

- la figure 2 représente l'enveloppe du dispositif d'assistance de la figure 2, l'enveloppe comportant des organes d'actionnement annulaires autour d'un axe d'enveloppe et déplaçables les uns par rapport aux autres en translation selon l'axe d'enveloppe,

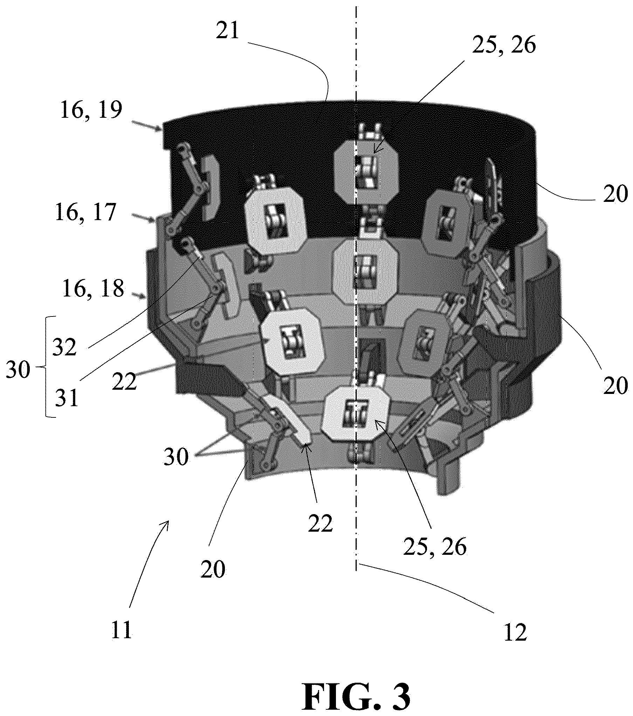

- la figure 3 représente une vue en coupe selon l'orientation référencée III-III sur la figure 2 de l'enveloppe de la figure 2, chaque organe d'actionnement comportant un corps annulaire et des plaquettes sur lesquelles une surface de contact des organes d'actionnement avec la surface extérieure du muscle cardiaque est réparties,

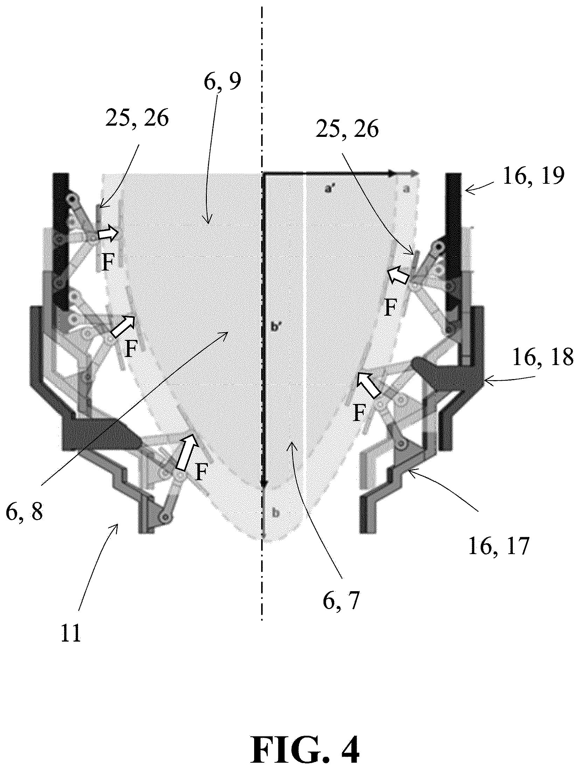

- la figure 4 représente schématiquement des étapes d'un procédé d'assistance mettant en œuvre le dispositif d'assistance de la figure 1, les organes d'actionnement étant déplacés selon l'axe d'enveloppe par le système de contrôle pour faire varier le volume du muscle cardiaque entre un volume diastolique et un volume systolique.

### DESCRIPTION DÉTAILLÉE

La figure 1 représente un dispositif d'assistance 10 du muscle cardiaque 1, ou myocarde, d'un être vivant, en particulier d'un patient, souffrant d'une pathologie, en particulier d'une insuffisance cardiaque.

Le dispositif d'assistance 10 est configuré comme un exo-muscle implantable réalisant une contention active du muscle cardiaque 1. Le dispositif d'assistance 10 peut ainsi assister le muscle cardiaque 1 dans ses mouvements visant à faire varier son volume au cours d'un cycle cardiaque entre un volume diastolique, en diastole, et un volume systolique, en systole. Les volumes diastolique et systolique peuvent être déterminés de toute manière appropriée. Il peut notamment s'agir des volumes diastolique et systolique naturels du muscle cardiaque du patient à un stade préalable à la mise en place du dispositif d'assistance 10 et, le cas échéant, avant l'atteinte du muscle cardiaque 1 par la pathologie. Il peut également s'agir de volumes diastolique et systolique moyens définis à partir d'une population appartenant à la même catégorie que le patient. Il peut bien entendu s'agir de tous autres volumes diastolique et systolique jugés appropriés par le personnel soignant.

Le muscle cardiaque 1 présente un axe de muscle cardiaque 2 entre un apex 3 et une base 4, et une surface extérieure 5. Il comporte une pluralité de zones d'intérêt 6 identifiées par tout moyen approprié, et notamment dans la littérature, par des essais ou des observations, par imagerie médicale ou autre. Chaque zone d'intérêt 6 présente une surface extérieure déplaçable selon un débattement entre la diastole et la systole du cycle cardiaque. Les débattements des différentes zones d'intérêt 6 du muscle cardiaque 1 peuvent être déterminés de toute manière appropriée. Il peut notamment s'agir de débattements naturels du muscle cardiaque 1 du patient à un stade préalable à la mise en place du dispositif d'assistance 10 et, le cas échéant, avant l'atteinte du muscle cardiaque 1 par la pathologie. Il peut également s'agir de débattements moyens définis à partir d'une population appartenant à la même catégorie que le patient. Il peut bien entendu s'agir de tous autres débattements jugés appropriés par le personnel soignant.

En particulier, pour le mode de réalisation représenté, les zones d'intérêt 6 prises en considération sont adjacentes selon l'axe de muscle cardiaque 2 et comprennent une zone d'intérêt apicale 7, au niveau de l'apex 3, et une zone d'intérêt basale 9 au niveau de la base 4. Les zones d'intérêt 6 prises en considération comprennent également une zone d'intérêt intermédiaire 8 entre les zones d'intérêt apicale 7 et basale 9. Comme visible sur la figure 4, le débattement de la zone d'intérêt apicale 7 a une composante essentiellement axiale, identifiée par les références b et b', et le débattement de la zone d'intérêt basale 9 a une composante essentiellement radiale, identifiée par les références a et a'.

L'invention n'est pas limitée à un muscle cardiaque 1 segmenté en trois zones d'intérêt 6. Dans d'autres modes de réalisation, plus d'une zone d'intérêt intermédiaire 8 peut être prévue entre les zones d'intérêt apicale 7 et basale 9.

Sur les figures 1 et 2, le dispositif d' assistance 10 comprend une enveloppe 11 implantable présentant un axe d'enveloppe 12 destiné à être placé selon l'axe de muscle cardiaque 2.

L'enveloppe 11 comporte une pluralité d'organes d'actionnement 16 annulaires autour de l'axe d'enveloppe 12. Chaque organe d'actionnement 16 présente une surface de contact 25 configurée pour être placée en contact avec au moins une partie de la surface extérieure de l'une des zones d'intérêt 6. La pluralité d'organes d'actionnement 16 comporte alors au moins un organe d'actionnement apical 17 dont la surface de contact est configurée pour être placée en contact avec au moins une partie de la surface extérieure de la zone d'intérêt apicale 7, et un organe d'actionnement basal 19 dont la surface de contact est configurée pour être placée en contact avec au moins une partie de la surface extérieure de la zone d'intérêt basale 9. La pluralité d'organes d'actionnement 16 comporte également un organe d'actionnement intermédiaire 18 dont la surface de contact est configurée pour être placée en contact avec au moins une partie de la surface extérieure de la zone d'intérêt intermédiaire 8. Dans d'autres modes de réalisation, le nombre d'organe d'actionnement intermédiaire 18 est adapté en fonction du nombre de zone d'intérêt intermédiaire 8.

Sur la figure 3, chaque organe d'actionnement 16 comporte un corps 20 annulaire autour de l'axe d'enveloppe 12 et présentant une surface intérieure 21. Les organes d'actionnement 16 sont imbriqués les uns dans les autres. En particulier, ils peuvent être pris en considération par paire d'organes d'actionnement comportant un organe d'actionnement externe et un organe d'actionnement interne. Dans chaque paire d'organes d'actionnement, le corps 20 de l'organe d'actionnement interne s'étend en partie dans le corps 20 de l'organe d'actionnement externe. Dans le mode de réalisation représenté, l'organe d'actionnement intermédiaire 18 et l'organe d'actionnement apical 17 forment une paire d'organes d'actionnement dans laquelle l'organe d'actionnement intermédiaire 18 est l'organe d'actionnement externe et l'organe d'actionnement apical 17 est l'organe d'actionnement interne. L'organe d'actionnement apical 17 et l'organe d'actionnement basal 19 forment une autre paire d'organes d'actionnement dans laquelle l'organe d'actionnement apical 17 est l'organe d'actionnement externe et l'organe d'actionnement basal 19 est l'organe d'actionnement interne.

Dans le mode de réalisation représenté, l'organe d'actionnement apical 17 présente une dimension axiale, selon l'axe d'enveloppe 12, supérieure à la dimension axiale de l'organe d'actionnement intermédiaire 18.

Sur la surface intérieure 21 du corps 20, chaque organe d'actionnement 16 comporte en outre une pluralité de plaquettes 22 réparties et présentant des surfaces élémentaires de contact 26 opposées à la surface intérieure 21 du corps 20. Les surfaces élémentaires de contact 26 des plaquettes 22 forment la surface de contact 25 de l'organe d'actionnement 16.

La surface élémentaire de contact 26 de chacune des plaquettes 22 peut être déplaçable par rapport à la surface intérieure 21 du corps 20 de l'organe d'actionnement 16 et, en particulier, orientable pour assurer un contact intime et permanent avec la surface extérieure 5 du muscle cardiaque 1.

Dans le mode de réalisation représenté, chacune des plaquettes 22 peut être montée sur la surface intérieure 21 du corps 20 de l'un des organes d'actionnement 16 par l'intermédiaire d'un agencement de biellettes 30. L'agencement de biellettes 30 comprend une première biellette 31 présentant des extrémités opposées articulées respectivement sur la plaquette 22 et sur la surface intérieure 21 du corps 20 de l'organe d'actionnement 16. L'agencement de biellettes 30 comprend également une deuxième biellette 32 présentant des extrémités opposées articulées respectivement sur la plaquette 22 et sur la surface intérieure 21 du corps 20 de l'organe d'actionnement adjacent selon l'axe d'enveloppe 12, à savoir l'organe d'actionnement apicale 17 pour les plaquettes 22 de l'organe d'actionnement basal 19 et l'un des organes d'actionnement intermédiaire 18 et basal 19 pour l'organe d'actionnement apical 17. En variante, chaque agencement de biellettes pourrait comprendre plusieurs premières biellettes 31 et/ou plusieurs deuxièmes biellettes 32.

Outre l'enveloppe 11, le dispositif d'actionnement 10 comprend un système de contrôle 35 représenté sur la figure 1.

Sur la figure 4, le système de contrôle 35 comprend un ou plusieurs actionneurs 36 configurés pour déplacer chacune des surfaces de contact 25 selon une course correspondant à au moins une partie du débattement de la zone d'intérêt 6 avec laquelle la surface de contact 25 est en contact.

Dans le mode de réalisation représenté, le système de contrôle comprend au moins un actionneur principal 37, notamment électromagnétique ou piézoélectrique, configuré pour déplacer les corps 20 des organes d'actionnement 16 les uns par rapport aux autres en translation selon l'axe d'enveloppe 12. Du fait des agencements de biellettes 30, le déplacement des organes d'actionnement 16 selon l'axe d'enveloppe 12 induit un déplacement des plaquettes 22 par rapport aux surfaces intérieures 21 des corps 20 de l'organe d'actionnement 16. En particulier, la course de la surface de contact 25 de l'organe d'actionnement apical 17 est essentiellement axiale selon au moins une partie de la composante essentiellement axiale b - b' du débattement de la zone d'intérêt apicale 7. Par ailleurs, la course de la surface de contact 25 de l'organe d'actionnement basal 19 est essentiellement radiale selon au moins une partie de la composante essentiellement radiale a - a' du débattement de la zone d'intérêt basale 9.

L'enveloppe 11 peut alors présenter un état déployé dans lequel les organes d'actionnement 16 sont éloignés les uns des autres et les surfaces de contact 25 délimitent le volume diastolique du muscle cardiaque 1, et un état rétracté dans lequel les organes d'actionnement 16 sont rapprochés les uns des autres et les surfaces de contact 25 délimitent le volume systolique du muscle cardiaque 1. Dans le mode de réalisation représenté, les organes d'actionnement 16 sont imbriqués les uns dans les autres dans l'état rétracté et dans l'état déployé de l'enveloppe 12.

Ces dispositions permettent de respecter la physionomie de la contraction du muscle cardiaque 1 en appliquant des efforts, illustrés par des flèches F sur la figure 4, répartis sur l'ensemble des zones d'intérêt 6 selon des orientations conformes à une convergence naturelle des efforts du muscle cardiaque 1 pour successivement aspirer et éjecter des volumes de sang nécessaires.

Le ou les actionneurs 36 peuvent être activables électriquement et le système de contrôle 35 peut comprendre une source d'alimentation en énergie électrique connectée aux actionneurs 36. La source d'alimentation en énergie électrique peut alors comprendre au moins une batterie 41 implantable.

La batterie 41 peut également être intégrée à une unité implantable 40 du système de contrôle 35 comportant :
- une mémoire 42 dans laquelle est enregistré un logiciel de pilotage,
- une carte de puissance 43 connectée aux actionneurs 36, et
- un microcontrôleur 44 connecté à la mémoire 42 et à la carte de puissance, et configuré pour activer la carte de puissance 43 conformément à des instructions du logiciel de pilotage pour actionner les actionneurs de manière appropriée.

A cet égard, le dispositif d'assistance 10 peut être synchronisé sur l'activité du cœur caractérisée par une pluralité de paramètres d'activité. Le système de contrôle 35 peut alors comprendre un dispositif de détection 45 configuré pour détecter un ou plusieurs des paramètres d'activité et pour activer les actionneurs à partir des paramètres d'activité détectés.

Le système de contrôle 35 peut alors comprendre une interface de communication 46 bidirectionnelle configurée pour recevoir de nouvelles instructions ou des mises à jour du logiciel de pilotage et pour envoyer des données mesurées par l'unité implantable vers un serveur distant 50.

Le dispositif d'assistance 10 tel que décrit ci-dessus peut être mis en œuvre dans un procédé d'assistance prévoyant d'implanter l'enveloppe 11 sur le muscle cardiaque 1, la surface de contact 25 de chacun des organes d'actionnement 16 étant placée en contact avec au moins une partie de la surface extérieure de la zone d'intérêt 6 correspondante du muscle cardiaque 1. L'organe d' actionnement intermédiaire 18 est par exemple configuré pour être solidarisé par tout type de système d'ancrage approprié au muscle cardiaque 1. Une fois l'enveloppe 11 implantée, le procédé d'assistance se poursuit en déplaçant, le cas échéant en coordination avec l'activité du cœur, les organes d'actionnement 16 selon l'axe d'enveloppe 12, en particulier en translation, entre l'état déployé de l'enveloppe 11 dans lequel les organes d'actionnement 16 sont éloignés les uns des autres et les surfaces de contact 25 délimitent le volume diastolique du muscle cardiaque 1, et l'état rétracté de l'enveloppe 11 dans lequel les organes d'actionnement 16 sont rapprochés les uns des autres et les surfaces de contact 25 délimitent le volume systolique du muscle cardiaque 1. En particulier, chacune des surfaces de contact 25 est déplacée selon une course correspondant à au moins une partie du débattement de la zone d'intérêt 6 avec laquelle elle est en contact. Dans le mode de réalisation représenté, les organes d'actionnement apical 17 et basal 19 sont déplacés par rapport à l'organe d'actionnement intermédiaire 18.

La description a été faite avec des zones d'intérêt 6 se succédant selon l'axe de muscle cardiaque 2 et des organes d'actionnement 16 annulaires et déplacés intégralement en translation selon l'axe d'enveloppe 12. L'invention s'applique toutefois à tout autre agencement de zones d'intérêt 6, toute autre conformation et tout autre déplacement appropriés des organes d'actionnement 16 selon l'axe d'enveloppe 12. Pourvu que ces agencements soient couverts par les revendications attachées Par exemple, une partie seulement de chacun des organes d'actionnement 16 peut être déplacée selon l'axe d'enveloppe 12. Le déplacement selon l'axe d'enveloppe 12 peut être combiné avec un autre déplacement, notamment une rotation autour de l'axe d'enveloppe 12.

En outre, l'agencement relatif des organes d'actionnement 16 entre l'état rétracté et l'état déployé de l'enveloppe 12 pourrait être différent, les organes d'actionnement 16 n'étant imbriqués les uns dans les autres que dans l'état rétracté de l'enveloppe 12. En variante, les organes d'actionnement 16 pourraient ne pas être imbriqués et être à distance dans l'état rétracté comme dans l'état déployé, leur déplacement relatif faisant varier cette distance.

Dans d'autres modes de réalisation, qui ne font pas partie de l'invention revendiquée, en remplacement des agencements de biellettes 30. le système de contrôle 35 pourrait comprendre des actionneurs locaux, notamment électromagnétiques ou piézoélectriques, configurés chacun pour déplacer l'une des plaquettes 22 par rapport à la surface intérieure 21 du corps 20 de l'organe d'actionnement 16.

Selon d'autres modes de réalisation, la surface de contact 25 de chacun des organes d'actionnement 16 pourrait être configurée de toute autre manière appropriée, notamment directement sur la surface intérieure 21 du corps 20.

## Revendications

1. Dispositif d'assistance (10) du muscle cardiaque (1) d'un être vivant, le muscle cardiaque (1) présentant un axe de muscle cardiaque (2) entre un apex (3) et une base (4), et une surface extérieure (5), le muscle cardiaque (1) ayant un volume variable au cours d'un cycle cardiaque entre un volume diastolique et un volume systolique, le dispositif d'assistance (10) comprenant :
- une enveloppe (11) implantable présentant un axe d'enveloppe (12) destiné à être placé selon l'axe de muscle cardiaque (2), l'enveloppe (11) comportant une pluralité d'organes d'actionnement (16) annulaires autour de l'axe d'enveloppe (12), chaque organe d'actionnement (16) présentant une surface de contact (25) configurée pour être placée en contact avec au moins une partie de la surface extérieure (5) du muscle cardiaque (1), le dispositif d'assistance (10) du muscle cardiaque (1) étant **caractérisé par**
- un système de contrôle (35) comprenant au moins un actionneur (36) configuré pour déplacer les organes d'actionnement (16) selon l'axe d'enveloppe (12) de telle manière que l'enveloppe (11) présente un état déployé dans lequel les organes d'actionnement (16) sont éloignés les uns des autres et les surfaces de contact (25) délimitent le volume diastolique du muscle cardiaque, et un état rétracté dans lequel les organes d'actionnement (16) sont rapprochés les uns des autres et les surfaces de contact (25) délimitent le volume systolique du muscle cardiaque (1).

2. Dispositif d'assistance (10) selon la revendication 1, le muscle cardiaque (1) présentant une pluralité de zones d'intérêt (6) adjacentes selon l'axe de muscle cardiaque (2), chaque zone d'intérêt (6) présentant une surface extérieure déplaçable selon un débattement entre une systole et une diastole du cycle cardiaque, dans lequel la surface de contact (25) de chacun des organes d'actionnement (16) est configurée pour être placée en contact avec au moins une partie de la surface extérieure de l'une des zones d'intérêt (6), et dans lequel ledit au moins un actionneur (36) est configuré pour déplacer chacune des surfaces de contact (25) selon une course correspondant à au moins une partie du débattement de la zone d'intérêt (6) avec laquelle ladite surface de contact (25) est en contact.

3. Dispositif d'assistance (10) selon la revendication 2, la pluralité de zones d'intérêt (6) du muscle cardiaque (1) présentant au moins une zone d'intérêt apicale (7) et une zone d'intérêt basale (9), le débattement de la zone d'intérêt apicale (7) ayant une composante essentiellement axiale et le débattement de la zone d'intérêt basale ayant une composante essentiellement radiale, dans lequel la pluralité d'organes d'actionnement (16) comporte au moins un organe d'actionnement apical (17) dont la surface de contact (25) est configurée pour être placée en contact avec au moins une partie de la surface extérieure de la zone d'intérêt apicale (7), et un organe d'actionnement basal (19) dont la surface de contact (25) est configurée pour être placée en contact avec au moins une partie de la surface extérieure de la zone d'intérêt basale (9), la course de la surface de contact (25) de l'organe d'actionnement apical (17) étant essentiellement axiale selon au moins une partie de la composante essentiellement axiale du débattement de la zone d'intérêt apicale (7), la course de la surface de contact (25) de l'organe d'actionnement basal (19) étant essentiellement radiale selon au moins une partie de la composante essentiellement radiale du débattement de la zone d'intérêt basale (9).

4. Dispositif d'assistance (10) selon l'une quelconque des revendication 1 à 3, dans lequel chaque organe d'actionnement (16) comporte un corps (20) annulaire autour de l'axe d'enveloppe (12) et présentant une surface intérieure (21), et une pluralité de plaquettes (22) réparties sur la surface intérieure (21) du corps (20), les plaquettes (22) présentant des surfaces élémentaires de contact (26) opposées à la surface intérieure (21) du corps (20), les surfaces élémentaires de contact (26) des plaquettes (22) formant la surface de contact (25) de l'organe d'actionnement (16).

5. Dispositif d'assistance (10) selon la revendication 4, dans lequel ledit au moins un actionneur (36) comprend au moins un actionneur principal (37), notamment électromagnétique ou piézoélectrique, configuré pour déplacer les corps (20) des organes d'actionnement (16) les uns par rapport aux autres selon l'axe d'enveloppe (12).

6. Dispositif d'assistance (10) selon l'une quelconque des revendications 4 et 5, dans lequel la surface élémentaire de contact (26) de chacune des plaquettes (22) est déplaçable par rapport à la surface intérieure (22) du corps (20) de l'organe d'actionnement (16).

7. Dispositif d'assistance (10) selon la revendication 6, dans lequel chacune des plaquettes (20) est montée sur la surface intérieure (21) du corps (20) de chacun des organes d'actionnement (16) par l'intermédiaire d'un agencement de biellettes (30) comprenant une première biellette (31) présentant des extrémités opposées articulées respectivement sur ladite plaquette (22) et sur ladite surface intérieure (21) du corps (20) de l'organe d'actionnement (16), et au moins une deuxième biellette (32) présentant des extrémités opposées articulées respectivement sur ladite plaquette (22) et sur la surface intérieure (21) du corps (20) de l'organe d'actionnement (16) adjacent selon l'axe d'enveloppe (12), de sorte que le déplacement des organes d'actionnement (16) selon l'axe d'enveloppe (12) induise un déplacement de la plaquette (22) par rapport à la surface intérieure (21) du corps (20) de l'organe d'actionnement (16).

8. Dispositif d'assistance (10) selon la revendication 6, dans lequel ledit au moins un actionneur (36) comprend au moins un actionneur local, notamment électromagnétique ou piézoélectrique, configuré pour déplacer la plaquette (22) par rapport à la surface intérieure (21) du corps (20) de l'organe d'actionnement (16).

9. Dispositif d'assistance (10) selon l'une quelconque des revendication 4 à 8, dans lequel la pluralité d'organes d'actionnement (16) comprend au moins une paire d'organes d'actionnement comportant un organe d'actionnement externe et un organe d'actionnement interne, le corps (20) de l'organe d'actionnement interne s'étendant en partie dans le corps (20) de l'organe d'actionnement externe au moins dans l'état rétracté de l'enveloppe (11).

10. Dispositif d'assistance (10) selon l'une quelconque des revendications 1 à 9, le muscle cardiaque (1) présentant une pluralité de paramètres d'activité, dans lequel le système de contrôle (35) comprend un dispositif de détection (45) configuré pour détecter au moins l'un des paramètres d'activité et pour activer ledit au moins un actionneur (36) à partir dudit au moins un paramètre d'activité détecté.

11. Dispositif d'assistance (10) selon l'une quelconque des revendications 1 à 10, dans lequel ledit au moins un actionneur (36) est activable électriquement et le système de contrôle (36) comprend une source d'alimentation en énergie électrique connectée audit au moins un actionneur (36).

12. Dispositif d'assistance (10) selon la revendication 11, dans lequel la source d'alimentation en énergie électrique comprend au moins une batterie (41) implantable.

## Patentansprüche

1. Unterstützungsvorrichtung (10) für den Herzmuskel (1) eines Lebewesens, wobei der Herzmuskel (1) eine Herzmuskelachse (2) zwischen einem Apex (3) und einer Basis (4) und eine Außenoberfläche (5) aufweist, wobei der Herzmuskel (1) im Laufe eines Herzzyklus ein variables Volumen zwischen einem diastolischen Volumen und einem systolischen Volumen aufweist, wobei die Unterstützungsvorrichtung (10) umfasst:
- eine implantierbare Hülle (11), die eine Hüllachse (12) aufweist, die dazu bestimmt ist, entlang der Herzmuskelachse (2) platziert zu werden, wobei die Hülle (11) eine Vielzahl von ringförmigen Betätigungselementen (16) um die Hüllachse (12) beinhaltet, wobei jedes Betätigungselement (16) eine Kontaktoberfläche (25) aufweist, die konfiguriert ist, um mit mindestens einem Teil der Außenoberfläche (5) des Herzmuskels (1) in Kontakt platziert zu werden, wobei die Unterstützungsvorrichtung (10) für den Herzmuskel (1) **gekennzeichnet ist durch**
- ein Steuersystem (35), das mindestens eine Betätigungsvorrichtung (36) umfasst, die konfiguriert ist, um die Betätigungselemente (16) entlang der Hüllachse (12) zu verschieben, sodass die Hülle (11) einen ausgeklappten Zustand, in dem die Betätigungselemente (16) voneinander entfernt sind und die Kontaktoberflächen (25) das diastolische Volumen des Herzmuskels begrenzen, und einen eingezogenen Zustand aufweist, in dem die Betätigungselemente (16) einander angenähert sind und die Kontaktoberflächen (25) das systolische Volumen des Herzmuskels (1) begrenzen.

2. Unterstützungsvorrichtung (10) nach Anspruch 1, wobei der Herzmuskel (1) eine Vielzahl von entlang der Herzmuskelachse (2) benachbarten Bereiche von Interesse (6) aufweist, wobei jeder Bereich von Interesse (6) eine äußere Oberfläche aufweist, die sich in einer Auslenkung zwischen einer Systole und einer Diastole des Herzzyklus verschieben lässt, wobei die Kontaktoberfläche (25) eines jeden der Betätigungselemente (16) konfiguriert ist, um mit mindestens einem Teil der äußeren Oberfläche eines der Bereiche von Interesse (6) in Kontakt platziert zu werden, und wobei die mindestens eine Betätigungsvorrichtung (36) konfiguriert ist, um jede der Kontaktoberflächen (25) um einen Weg zu verschieben, der mindestens einem Teil der Auslenkung des Bereichs von Interesse (6) entspricht, mit dem die Kontaktoberfläche (25) in Kontakt ist.

3. Unterstützungsvorrichtung (10) nach Anspruch 2, wobei die Vielzahl von Bereichen von Interesse (6) des Herzmuskels (1) mindestens einen apikalen Bereich von Interesse (7) und einen basalen Bereich von Interesse (9) aufweisen, wobei die Auslenkung des apikalen Bereichs von Interesse (7) eine im Wesentlichen axiale Komponente aufweist und die Auslenkung des basalen Bereichs von Interesse eine im Wesentlichen radiale Komponente aufweist, wobei die Vielzahl von Betätigungselementen (16) mindestens ein apikales Betätigungselement (17) beinhaltet, dessen Kontaktoberfläche (25) konfiguriert ist, um mit mindestens einem Teil der Außenoberfläche des apikalen Bereichs von Interesse (7) in Kontakt platziert zu werden, und ein basales Betätigungselement (19), dessen Kontaktoberfläche (25) konfiguriert ist, um mit mindestens einem Teil der Außenoberfläche des basalen Bereichs von Interesse (9) in Kontakt platziert zu werden, wobei der Weg der Kontaktoberfläche (25) des apikalen Betätigungselements (17) im Wesentlichen axial entlang mindestens eines Teils der im Wesentlichen axialen Komponente der Auslenkung des apikalen Bereichs von Interesse (7) ist, wobei der Weg der Kontaktoberfläche (25) des basalen Betätigungselements (19) im Wesentlichen radial entlang mindestens eines Teils der im Wesentlichen radialen Komponente der Auslenkung des basalen Bereichs von Interesse (9) ist.

4. Unterstützungsvorrichtung (10) nach einem der Ansprüche 1 bis 3, wobei jedes Betätigungselement (16) einen ringförmigen Körper (20) um die Hüllachse (12) beinhaltet und eine Innenoberfläche (21) aufweist, und eine Vielzahl von auf der Innenoberfläche (21) des Körpers (20) verteilten Plättchen (22), wobei die Plättchen (22) elementare Kontaktoberflächen (26) gegenüber der Innenoberfläche (21) des Körpers (20) aufweisen, wobei die elementaren Kontaktoberflächen (26) der Plättchen (22) die Kontaktoberfläche (25) des Betätigungselements (16) bilden.

5. Unterstützungsvorrichtung (10) nach Anspruch 4, wobei die mindestens eine Betätigungsvorrichtung (36) mindestens eine insbesondere elektromagnetische oder piezoelektrische Hauptbetätigungsvorrichtung (37) umfasst, die konfiguriert ist, um die Körper (20) der Betätigungselemente (16) entlang der Hüllachse (12) zueinander zu verschieben.

6. Unterstützungsvorrichtung (10) nach einem der Ansprüche 4 und 5, wobei die elementare Kontaktoberfläche (26) jedes der Plättchen (22) in Bezug auf die Innenoberfläche (22) des Körpers (20) des Betätigungselements (16) verschiebbar ist.

7. Unterstützungsvorrichtung (10) nach Anspruch 6, wobei jedes der Plättchen (20) an der Innenoberfläche (21) des Körpers (20) jedes der Betätigungselemente (16) anhand einer Schwingarmnanordnung (30) montiert ist, die einen ersten Schwingarm (31) umfasst, die jeweils gegenüberliegende Enden aufweist, die an dem Plättchen (22) und an der Innenoberfläche (21) des Körpers (20) des Betätigungselements (16) angelenkt sind, und mindestens einen zweiten Schwingarm (32), der jeweils an dem Plättchen (22) und an der Innenoberfläche (21) des Körpers (20) des benachbarten Betätigungselements (16) entlang der Hüllachse (12) angelenkte gegenüberliegende Enden aufweist, sodass die Verschiebung der Betätigungselemente (16) entlang der Hüllachse (12) eine Verschiebung des Plättchens (22) in Bezug zur Innenoberfläche (21) des Körpers (20) des Betätigungselements (16) bewirkt.

8. Unterstützungsvorrichtung (10) nach Anspruch 6, wobei die mindestens eine Betätigungsvorrichtung (36) mindestens eine lokale, insbesondere elektromagnetische oder piezoelektrische Betätigungsvorrichtung umfasst, die konfiguriert ist, um das Plättchen (22) in Bezug auf die Innenoberfläche (21) des Körpers (20) des Betätigungselements (16) zu verschieben.

9. Unterstützungsvorrichtung (10) nach einem der Ansprüche 4 bis 8, wobei die Vielzahl von Betätigungselementen (16) mindestens ein Betätigungselementepaar umfasst, das ein äußeres Betätigungselement und ein inneres Betätigungselement beinhaltet, wobei sich der Körper (20) des inneren Betätigungselements mindestens in dem eingezogenen Zustand der Hülle (11) teilweise in den Körper (20) des äußeren Betätigungselements erstreckt.

10. Unterstützungsvorrichtung (10) nach einem der Ansprüche 1 bis 9, wobei der Herzmuskel (1) eine Vielzahl von Aktivitätsparametern aufweist, wobei das Steuersystem (35) eine Erkennungsvorrichtung (45) umfasst, die konfiguriert ist, um mindestens einen der Aktivitätsparameter zu erkennen und die mindestens eine Betätigungsvorrichtung (36) ausgehend von dem mindestens einen erkannten Aktivitätsparameter zu aktivieren.

11. Unterstützungsvorrichtung (10) nach einem der Ansprüche 1 bis 10, wobei die mindestens eine Betätigungsvorrichtung (36) elektrisch aktivierbar ist und das Steuersystem (36) eine mit der mindestens einen Betätigungsvorrichtung (36) verbundene elektrische Energieversorgungsquelle umfasst.

12. Unterstützungsvorrichtung (10) nach Anspruch 11, wobei die elektrische Energieversorgungsquelle mindestens eine implantierbare Batterie (41) umfasst.

## Claims

1. Assistance device (10) for the heart muscle (1) of a living being, the heart muscle (1) having a heart muscle axis (2) between an apex (3) and a base (4), and an outer surface (5), the heart muscle (1) having a variable volume during a cardiac cycle between a diastolic volume and a systolic volume, the assistance device (10) comprising:
- an implantable envelope (11) having an envelope axis (12) intended to be placed along the heart muscle axis (2), the envelope (11) including a plurality of annular actuation members (16) around the envelope axis (12), each actuation member (16) having a contact surface (25) configured to be placed in contact with at least a portion of the outer surface (5) of the heart muscle (1), the assistance device (10) for the heart muscle (1) being **characterised by**
- a control system (35) comprising at least one actuator (36) configured to move the actuation members (16) along the envelope axis (12) in such a way that the envelope (11) has a deployed state in which the actuation members (16) are away from each other and the contact surfaces (25) delimit the diastolic volume of the heart muscle, and a retracted state in which the actuation members (16) are close to each other and the contact surfaces (25) delimit the heart-muscle systolic volume (1).

2. Assistance device (10) according to claim 1, the heart muscle (1) having a plurality of adjacent areas of interest (6) along the heart muscle axis (2), each area of interest (6) having an outer surface movable in a deflection between a systole and a diastole of the cardiac cycle, wherein the contact surface (25) of each of the actuation members (16) is configured to be placed in contact with at least a portion of the outer surface of one of the areas of interest (6), and wherein said at least one actuator (36) is configured to move each of the contact surfaces (25) in a travel corresponding to at least a portion of the movement of the area of interest (6) with which said contact surface (25) is in contact.

3. Assistance device (10) according to claim 2, the plurality of areas of interest (6) of the heart muscle (1) having at least one apical area of interest (7) and one basal area of interest (9), the movement of the apical area of interest (7) having an essentially axial component and the movement of the basal area of interest having an essentially radial component, wherein the plurality of actuation members (16) includes at least one apical actuation member (17) the contact surface (25) of which is configured to be placed in contact with at least a portion of the outer surface of the apical area of interest (7), and a basal actuation member (19) the contact surface (25) of which is configured to be placed in contact with at least a portion of the outer surface of the basal area of interest (9), the travel of the contact surface (25) of the apical actuation member (17) being essentially axial along at least a part of the essentially axial component of the movement of the apical area of interest (7), the travel of the contact surface (25) of the basal actuating member (19) being essentially radial along at least a part of the essentially radial component of the movement of the basal region of interest (9).

4. Assistance device (10) according to any one of claims 1 to 3, wherein each actuating member (16) includes an annular body (20) around the envelope axis (12) and having an inner surface (21), and a plurality of pads (22) distributed on the inner surface (21) of the body (20), the pads (22) having elementary contact surfaces (26) opposite the inner surface (21) of the body (20), the elementary contact surfaces (26) of the pads (22) forming the contact surface (25) of the actuating member (16).

5. Assistance device (10) according to claim 4, wherein said at least one actuator (36) comprises at least one main actuator (37), in particular electromagnetic or piezoelectric, configured to move the bodies (20) of the actuation members (16) relative to one another along the envelope axis (12).

6. Assistance device (10) according to any one of claims 4 and 5, wherein the elementary contact surface (26) of each of the pads (22) is movable with respect to the inner surface (22) of the body (20) of the actuating member (16).

7. Assistance device (10) according to claim 6, wherein each of the pads (20) is mounted on the inner surface (21) of the body (20) of each of the actuating members (16) by means of an arrangement of connecting rods (30) comprising a first connecting rod (31) having opposite ends articulated respectively on said pad (22) and on said inner surface (21) of the body (20) of the actuating member (16), and at least one second connecting rod (32) having opposite ends articulated respectively on said plate (22) and on the inner surface (21) of the body (20) of the adjacent actuating member (16) along the envelope axis (12), such that the movement of the actuating members (16) along the envelope axis (12) causes a movement of the pad (22) relative to the inner surface (21) of the body (20) of the actuating member (16).

8. Assistance device (10) according to claim 6, wherein said at least one actuator (36) comprises at least one local actuator, in particular electromagnetic or piezoelectric, configured to move the pad (22) relative to the inner surface (21) of the body (20) of the actuating member (16).

9. Assistance device (10) according to any one of claims 4 to 8, wherein the plurality of actuation members (16) comprises at least one pair of actuation members including an external actuation member and an internal actuation member, the body (20) of the internal actuation member extending partially into the body (20) of the external actuation member at least in the retracted state of the envelope (11).

10. Assistance device (10) according to any one of claims 1 to 9, the heart muscle (1) having a plurality of activity parameters, wherein the control system (35) comprises a detection device (45) configured to detect at least one of the activity parameters and to activate said at least one actuator (36) using said at least one detected activity parameter.

11. Assistance device (10) according to any one of claims 1 to 10, wherein said at least one actuator (36) is electrically activatable and the control system (36) comprises an electrical energy supply connected to said at least one actuator (36).

12. Assistance device (10) according to claim 11, wherein the electrical energy supply source comprises at least one implantable battery (41).
